(19) Europäisches Patentamt
European Patent Office
Office européen des brevets

(11) Veröffentlichungsnummer: **0 688 755 A1**

# (12) EUROPÄISCHE PATENTANMELDUNG

(21) Anmeldenummer: **95109012.5**

(22) Anmeldetag: **12.06.95**

(51) Int. Cl.[6]: **C07C 43/11**, C07C 41/03, C11D 1/722

(30) Priorität: **21.06.94 DE 4421576**

(43) Veröffentlichungstag der Anmeldung: **27.12.95 Patentblatt 95/52**

(84) Benannte Vertragsstaaten: **AT BE CH DE DK ES FR GB LI NL PT SE**

(71) Anmelder: **HOECHST AKTIENGESELLSCHAFT**
**Brüningstrasse 50**
**D-65929 Frankfurt am Main (DE)**

(72) Erfinder: **Wimmer, Ignaz**
**Burger Str. 26**
**D-84543 Winhöring (DE)**
Erfinder: **Wehle, Detlef, Dr.**
**Schubertstr. 16**
**D-65611 Brechen (DE)**
Erfinder: **Crass, Gerhard, Dr.**
**Eichelsgartenstr. 9**
**D-61169 Friedberg (DE)**

## (54) Alkoxylierungsprodukf-Mischungen mit enger Alkoxylverteilung

(57) Die Erfindung betrifft Alkoxylierungsprodukt-Mischungen der Formel I mit enger Alkoxylverteilung,

$$R\left[(CH_2-CH_2-O)_x-(\underset{}{\overset{CH_3}{\overset{|}{C}}}H-CH_2-O)_y-H\right]_m \qquad (I)$$

wobei R der Rest einer organischen Verbindung mit mindestens einem aktiven Wasserstoffatom, m eine ganze Zahl von 1 bis zur Zahl der aktiven Wasserstoffatome der organischen Verbindung ist, x und y jeweils die durchschnittliche Molzahl an Ethylenoxid- bzw. Propylenoxideinheiten sind und unabhängig voneinander eine ganze Zahl von 2 bis 10 sind und die einzelnen Alkoxylierungseinheiten der Alkoxylierungsprodukt-Mischung jeweils maximal einen Alkoxylierungsgrad von 18 Gew% aufweisen.

Weitere Gegenstände der Erfindung sind ein Verfahren zu deren Herstellung sowie deren Verwendung.

EP 0 688 755 A1

Die Erfindung bezieht sich auf Alkoxylierungsprodukt-Mischungen mit enger Alkoxylverteilung, ferner auf ein Verfahren zur Herstellung dieser Alkoxylierungsprodukt-Mischungen sowie auf ihre Verwendung.

Alkoxylate haben große Bedeutung, zum Beispiel als Zwischenprodukte für Derivatisierungen und als nichtionogene Komponenten in technischen und kosmetischen Wasch- und Reinigungsmitteln. Sie werden ferner in einer Vielzahl von Anwendungsgebieten als Emulgatoren, Dispergatoren und dergleichen eingesetzt. Dabei werden häufig solche Alkoxylate gewünscht, die eine enge Verteilung der Alkoxylierungshomologen aufweisen.

In der noch nicht veröffentlichten deutschen Patentanmeldung mit dem Aktenzeichen P 4341576.8 werden Ethoxylate mit enger Homologenverteilung beschrieben. Es wird ferner ein Verfahren zur Ethoxylierung von aktive Wasserstoffatome enthaltenden Verbindungen in Gegenwart spezieller Erdalkalimetallsalze von Alkyl- oder Alkenylbernsteinsäuremonoestern als Katalysatoren beschrieben.

In EP-A-0 133 715 werden Alkoxylierungsprodukt-Mischungen beschrieben, die durch Reaktion einer organischen Verbindung mit mindestens einem aktiven Wasserstoff-Atom mit einem Epoxid, wie Ethylenoxid oder Propylenoxid, erhalten werden, wobei die Produktmischung mindestens eine Alkoxylierungseinheit aufweist, die 20-40 Gew% der Mischung beträgt.

Es hat sich jedoch gezeigt, daß die aus dem Stand der Technik bekannten Alkoxylierungsprodukt-Mischungen nur begrenzt biologisch abgebaut werden. Im Hinblick auf ein gesteigertes Umweltbewußtsein ist aber gerade auf das Merkmal der biologischen Abbaubarkeit bei Detergenzien, Wasch- und Reinigungsmitteln ein besonderes Augenmerk gerichtet.

Es bestand somit die Aufgabe, Alkoxylierungsprodukt-Mischungen zur Verfügung zu stellen, die sich durch eine gute biologische Abbaubarkeit auszeichnen.

Es hat sich gezeigt, daß diese Aufgabe gelöst werden kann durch die Bereitstellung von Alkoxylierungsprodukt-Mischungen mit enger Alkoxylverteilung.

Die Erfindung betrifft Alkoxylierungsprodukt-Mischungen der Formel I mit enger Alkoxylverteilung,

$$R\left[(CH_2-CH_2-O)_x-(\overset{\displaystyle CH_3}{\overset{|}{C}H}-CH_2-O)_y\,H\right]_m \qquad (I)$$

wobei R der Rest einer organischen Verbindung mit mindestens einem aktiven Wasserstoffatom, m eine ganze Zahl von 1 bis zur Zahl der aktiven Wasserstoffatome der organischen Verbindung ist, x und y jeweils die durchschnittliche Molzahl an Ethylenoxid- bzw. Propylenoxideinheiten sind und unabhängig voneinander eine ganze Zahl von 2 bis 10 sind und die einzelnen Alkoxylierungseinheiten der Alkoxylierungsprodukt-Mischung jeweils maximal einen Alkoxylierungsgrad von 18 Gew% aufweisen.

In Formel I ist m bevorzugt eine Zahl von 1 bis 3 und besonders bevorzugt gleich 1. Die Werte x und y in Formel I sind unabhängig voneinander bevorzugt eine Zahl von 2 bis 6, y ist besonders bevorzugt eine Zahl von 2 bis 4.

Bevorzugt sind Alkoxylierungsprodukt-Mischungen der Formel I, in denen m = 1 ist und x und y unabhängig voneinander eine Zahl von 2 bis 6 sind.

Besonders bevorzugt sind Alkoxylierungsprodukt-Mischungen der Formel I, in denen m = 1 ist, x und y unabhängig voneinander sind und x eine Zahl von 2 bis 6 und y eine Zahl von 2 bis 4 ist.

Der Rest R in Formel I leitet sich von organischen Verbindungen mit mindestens einem aktiven Wasserstoffatom ab, beispielsweise von Hydroxylgruppen enthaltenden Verbindungen, Aminverbindungen und Säureverbindungen wie Fettsäuren.

Bevorzugt leitet sich der Rest R von Hydroxylgruppen enthaltenden Verbindungen ab, beispielsweise von Alkoholen, Aminoalkoholen, Perfluoralkylalkoholen, Glykolen, Glykolmonoethern, Phenolen, Kresolen, wobei Alkohole besonders bevorzugt sind. Sie können aus nativer Quelle oder aus Synthese-Prozessen stammen, geradkettig oder verzweigt, gesättigt oder ungesättigt, ein oder mehrwertig sein, beispielsweise Oxoalkohole oder Fettalkohole.

Insbesondere werden einwertige, geradkettige oder verzweigte, gesättigte oder ungesättigte $C_8$-$C_{18}$-Alkohole oder Gemische davon eingesetzt, zum Beispiel Gemische aus $C_{12}$ und $C_{14}$-Alkohol ($C_{12/14}$). Als spezielle Beispiele für die besonders bevorzugten Alkohole seien genannt: Nonanol, Isononylalkohol, Decanol, Undecanol, Isoundecanol, Laurylalkohol, Isotridecylalkohol, Stearylalkohol, Kokosfettalkohol und Gemische davon, ferner 2-Ethylhexanol, 2-Hexyldecanol und 2-Octyldecanol.

In Formel I ist R demzufolge besonders bevorzugt eine einwertige, geradkettige oder verweigte, gesättigte

oder ungesättigte $C_8$-$C_{18}$-Alkoxygruppe oder Gemische davon.

Bevorzugt sind daher Alkoxylierungsprodukte der Formel I, in denen m = 1 ist, x und y unabhängig voneinander eine Zahl von 2 bis 6 sind und R eine einwertige, geradkettige oder verzweigte, gesättigte oder ungesättigte $C_8$-$C_{18}$-Alkoxygruppe oder Gemische davon bedeutet.

Besonders bevorzugt sind Alkoxylierungsprodukte der Formel I, in denen m = 1 ist, x und y unabhängig voneinander sind, x eine Zahl von 2 bis 6 und y eine Zahl von 2 bis 4 ist und R eine einwertige, geradkettige oder verzweigte, gesättigte oder ungesättigte $C_8$-$C_{18}$-Alkoxygruppe oder Gemische davon bedeutet.

Gegenstand der Erfindung ist ferner ein Verfahren zur Herstellung von Alkoxylierungsprodukt-Mischungen gemäß der Formel I umfassend die Verfahrensschritte

- Umsetzung einer organischen Verbindung mit mindestens einem aktiven Wasserstoffatom mit Ethylenoxid in einem Molverhältnis von 2 bis 10 mol Ethylenoxid pro mol aktiven Wasserstoffs in der zu ethoxylierenden Verbindung in Gegenwart von mindestens einem Erdalkalimetallsalz der Alkyl- oder Alkenylbernsteinsäuremonoester der nachstehenden Formeln (II) und (III) als Katalysator

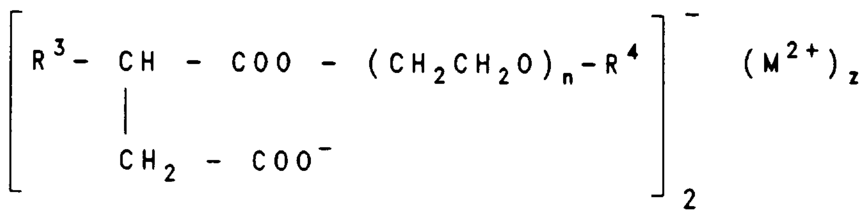

(II)

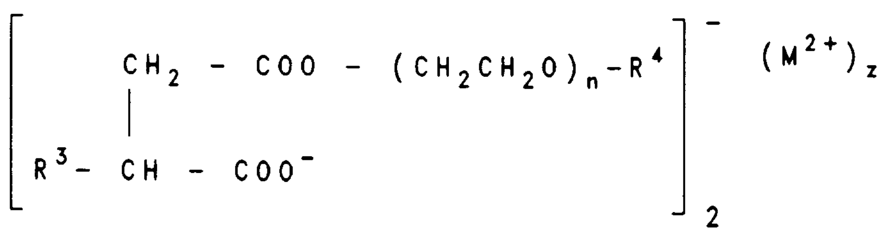

(III)

wobei

| | |
|---|---|
| $R^3$ | $C_8$ bis $C_{30}$-Alkyl oder $C_8$ bis $C_{30}$-Alkenyl, |
| n | eine Zahl von 0 bis 6, |
| $R^4$ | $C_1$ bis $C_{18}$-Alkyl oder $C_3$ bis $C_{18}$-Alkenyl oder Wasserstoff, wenn n = 1 oder > 1 ist, |
| M | Ba, Ca oder Sr und |
| z | eine Zahl von 0,9 bis 1,8 ist, |

- anschließend Zugabe einer Base zu der erhaltenen Ethoxylatprodukt-Mischung und Zugabe von Propylenoxid.

Die Umsetzung der organischen Verbindung mit mindestens einem aktiven Wasserstoffatom mit Ethylenoxid in Gegenwart von mindestens einem Erdalkalisalz der Alkyl- oder Alkenylbernsteinsäurehalbester wird üblicherweise bei einer Temperatur von 60 bis 200 °C, vorzugsweise 100 bis 180 °C, und einem Druck von etwa 0,5 bis 6 bar durchgeführt, wobei das Ethylenoxid portionsweise oder kontinuierlich zudosiert wird. Die zugegebene Menge an Ethylenoxid liegt bei 2 bis 10 mol, vorzugsweise bei 2 bis 8 mol und insbesondere bei 2 bis 6 mol pro mol aktiven Wasserstoffs in der zu ethoxylierenden Verbindung. Die erhaltene Ethoxylierungsprodukt-Mischung kann im allgemeinen ohne vorherige Abtrennung des Katalysators weiter umgesetzt werden.

Die als Katalysator zu verwendenden Erdalkalisalze von Alkyl- und/oder Alkenylbernsteinsäurehalbestern (hierbei handelt es sich um Stellungsisomere, die im allgemeinen in Mischung vorliegen) sind bekannt und im Handel erhältlich, zum Beispiel als ®HOSTACOR (® = eingetragenes Warenzeichen von Hoechst). In den Formeln (II) und (III) ist $R^3$ vorzugsweise ein $C_9$ bis $C_{20}$-Alkyl oder ein $C_9$ bis $C_{20}$-Alkenyl, n ist vorzugsweise ein Zahl von 0 bis 3, $R^1$ ist vorzugsweise ein $C_1$ bis $C_{12}$-Alkyl oder ein $C_3$ bis $C_{12}$-Alkenyl und kann vorzugsweise auch das Wasserstoffatom sein, wenn n = 1 oder >1 ist und z ist vorzugsweise eine Zahl von 1 bis 1,3 und besonders bevorzugt 1. M ist aus Zweckmäßigkeitsgründen vorzugsweise Ca. Für z<1 können neben M noch Protonen als weitere Gegenionen vorhanden sein. Die Alkenylgruppen haben vorzugsweise 1 bis 3 Doppelbindungen. Die Alkyl- und Alkenylgruppen können gerade oder verzweigt sein. Alkyl und Alkenyl können auch in Form von Gemischen vorliegen, zum Beispiel in Form

eines Gemisches aus $C_{12}$ und $C_{14}$-Alkyl ($C_{12/14}$-Alkyl) oder $C_{12}$ und $C_{14}$-Alkenyl ($C_{12/14}$-Alkenyl) oder aus $C_{12}$ und $C_{14}$-Alkyl- und -Alkenylgruppen. Von den zu verwendenden Erdalkalisalzen des mit einer Alkylgruppe oder einer Alkenylgruppe substituierten Bernsteinsäurehalbesters sind die mit einer Alkenylgruppe substituierten bevorzugt, das heißt, $R^3$ in den Formeln (II) und (III) ist vorzugsweise eine der genannten Alkenylgruppen. Der Rest $R^4$ ist dagegen vorzugsweise eine der genannten Alkylgruppen. Beispiele für Alkyl- und Alkenylreste sind also Methyl, Propyl, Butyl, Iso-butyl, Octyl, Octenyl, Decyl, Decenyl, Dodecyl (Lauryl), Dodecenyl, Oleyl, Octadecadienyl, Octadecatrienyl und Talgfettalkyl.

Es wurde gefunden, daß eine noch höhere Wirksamkeit hinsichtlich katalytischer Aktivität und enger Homologenverteilung erreicht wird, wenn man bei der Ethoxylierung in Gegenwart von mindestens einem Bernsteinsäuremonoestersalz der Formeln (II) und (III) arbeitet, von dem 10 bis 70 %, vorzugsweise 30 bis 60 %, seiner titrierbaren Alkalität neutralisiert worden sind mit einer anorganischen Säure, die mit den Kationen Ba, Ca und Sr in Wasser schwerlösliche Salze bildet. Bevorzugte Mineralsäuren sind Schwefelsäure ($H_2SO_4$) und schwefelige Säure ($H_2SO_3$) sowie Phosphorsäure ($H_3PO_4$) und phosphorige Säure ($H_3PO_3$).

Die bei der Ethoxylierung einzusetzende Menge des Erdalkalisalzes der Alkyl- oder Alkenylbernsteinsäurehalbester kann in weiten Grenzen variieren und liegt im allgemeinen bei 0,1 bis 5 Gew.-%, vorzugsweise 0,5 bis 3 Gew.-%, bezogen auf das Gewicht der zu alkoxylierenden organischen Verbindung mit mindestens einem aktiven Wasserstoffatom. Der Katalysator wird der zu alkoxylierenden Verbindung in der angegebenen Menge zugesetzt. Er kann auch in situ erzeugt werden, zum Beispiel in der Weise, daß man zunächst der zu alkoxylierenden Verbindung eine definierte Menge von einem Barium-, Calcium- oder Strontiumoxid, -carbonat oder -hydroxid, wobei die Hydroxide bevorzugt sind, und die darauf abgestimmte, aus den Formeln (II) und (III) resultierende stöchiometrische Menge an substituierter Bernsteinsäureanhydridverbindung zusetzt und dann trocknet, gegebenenfalls unter vermindertem Druck, worauf mit der Ethoxylierung begonnen wird. Die in-situ-Variante kann auch die beschriebene Teilneutralisation umfassen.

Die Erdalkalisalze der Alkyl- oder Alkenylbernsteinsäurehalbester weisen eine hohe katalytische Aktivität auf und führen in relativ kurzer Reaktionszeit zu einem praktisch vollständigen Umsatz und zu hoher Ausbeute. Das Ethoxylat weist demzufolge eine enge Homologenverteilung und einen nur noch geringen Anteil der zu ethoxylierenden Ausgangsyerbindung auf.

Die nach der Ethoxylierung erhaltene Ethoxylatprodukt-Mischung wird zur weiteren Katalyse mit einer Base versetzt und das Gemisch anschließend gegebenenfalls getrocknet. Bevorzugt wird die Ethoxylatprodukt-Mischung mit einer wäßrigen Alkalimetallhydroxid-Lösung oder einem Alkalimetallalkanolat als Base versetzt. Als wäßrige Alkalimetallhydroxid-Lösung wird bevorzugt eine Natriumhydroxid-Lösung eingesetzt. Zum Einsatz kommen weiterhin Alkalimetallalkanolate von $C_1$-$C_4$- Alkoholen, bevorzugt Methanol oder Ethanol. Die Trocknung erfolgt üblicherweise bei einer Temperatur von 70-150 °C, vorzugsweise 80-120 °C, und unter einem vermindertem Druck von 10-100 mbar, vorzugsweise 10-30 mbar.
Die Menge der eingesetzten Base ist so bemessen, daß im rohen Endprodukt eine Alkalizahl von 1-5 resultiert.

Zu der gegebenenfalls getrockneten Ethoxylierungsprodukt-Mischung wird das Propylenoxid zugegeben. Üblicherweise erfolgt diese Zugabe über einen Zeitraum von 1-10 h, bei einer Temperatur von 100 - 150 °C, vorzugsweise 110 - 140 °C, wobei das Propylenoxid portionsweise oder kontinuierlich zudosiert wird. Die Menge an Propylenoxid liegt im allgemeinen bei 2 bis 10 mol, vorzugsweise bei 2 bis 6 mol und insbesondere bei 2 bis 4 mol pro mol aktiven Wasserstoffs in der zu alkoxylierender Verbindung. Gegebenenfalls wird die Alkoxylierungsprodukt-Mischung anschließend mit einer $C_1$-$C_{10}$-Carbonsäure neutralisiert, bevorzugt werden hierbei geradkettige, aliphatische $C_1$-$C_3$-Carbonsäuren und verzweigt, aliphatische $C_8$-$C_{10}$-Carbonsäuren verwendet. Die erhaltene Alkoxylierungsprodukt-Mischung kann im allgemeinen ohne vorherige Abtrennung der Katalysatoren weiterverwendet werden.

Die erfindungsgemäßen Alkoxylierungsprodukt-Mischungen besitzen eine enge Homologenverteilung und zeichnen sich durch eine besonders gute biologische Abbaubarkeit aus. Sie können in ihrer Eigenschaft als schwachschäumende Tenside in Reinigungsmitteln, beispielsweise Flüssigwaschmitteln, Flüssigreinigungsmitteln, Maschinengeschirrspülmitteln und Flaschenreinigungsmitteln eingesetzt werden. Sie können ferner als Dispergier- oder Netzmittel verwendet werden.

Die Erfindung wird nun an Beispielen näher erläutert.

Die Homologenverteilung der in den Beispielen erhaltenen Alkoholalkoxylate wurde nach vorausgehender Derivatisierung (Silylierung der OH-Endgruppen der Alkoholblockalkoxylate unter Einsatz von $(CH_3)_3SiCl$ ) mittels Kapillargaschromatographie bestimmt. Als Maß für die Homologenverteilung wird der sogenannte Q-Wert gemäß Gleichung $Q = n^* \cdot p^2$ angegeben, in der n* die durchschnittliche Zahl der an die organische Ausgangsverbindung mit mindestens einem aktiven Wasserstoffatom angelagerten Ethylenoxid- und Propylenoxidmoleküle (mittlerer Alkoxylierungsgrad) ist und p die Gewichtsprozente des gew%ig am

stärksten vorkommenden Alkoxylierungshomologen bedeutet. Dieser Q-Wert ist bekanntlich insbesondere dann ein guter Maßstab, wenn es sich um Alkoxylate mit einem im wesentlichen gleichen durchschnittlichen Alkoxylierungsgrad handelt. Höhere Werte von Q zeigen eine selektivere Alkoxylierung und ein Alkoxylat mit einer engeren Homologenverteilung an.

Die Bestimmung der biologischen Abbaubarkeit erfolgte im modifizierten Sturmtest gemäß der OECD-Richtlinie 301B, bei dem die Substanzen über einen Zeitraum von 29 Tagen an einem nicht adaptierten Belebtschlamm geprüft wurden.

Beispiele

Herstellung von $C_{12}$-$C_{14}$-Fettalkohol-Ethylenoxid/Propylenoxid-Blockalkoxylaten

Beispiel 1

1. Stufe:

In einem rührbaren Druckreaktor wurden 194 g (1,00 mol) eines $C_{12/14}$-Alkohol-Gemisches vorgelegt. Nach üblicher Spülung des Reaktionsraumes mit Stickstoff wurde auf 90 °C aufgeheizt und 1 Stunde unter vermindertem Druck gerührt, um den Wassergehalt auf <0,10 Gew% zu senken. Anschließend wurden 2,9 g des Katalysators 1 zugesetzt und bei 150 - 160 °C in einem Zeitraum von 5 Stunden insgesamt 251 g Ethylenoxid (5,7 mol) zudosiert.
Zur Abreaktion des Epoxids wurde 1 Stunde bei 150 - 160 °C nachgerührt.

2. Stufe:

Zu dem aus der 1. Stufe im Reaktor vorliegenden $C_{12/14}$-Alkohol-Ethoxylat wurden 2,4 g einer 50 %igen wäßrigen NaOH-Lösung zugegeben und anschließend 2 Stunden bei 90 °C unter einem verminderten Druck von 20 mbar getrocknet.
Anschließend wurden bei 120 - 130 °C in einem Zeitraum von 5 Stunden insgesamt 232 g Propylenoxid (4,0 mol) zudosiert.
Zur Abreaktion des Epoxids wurde 1 Stunde bei 135 °C nachgerührt.
Anschließend wurde mit Isononansäure neutralisiert.

Bei Katalysator 1 handelt es sich um eine Verbindung der Formel (II), wobei
$R^3$ iso-$C_{12}$-Alkenyl,
$R^4$ iso-Butyl,
n 0,
M Ca und
z 1 ist
und dieses Alkenylbernsteinsäurehalbester-Calciumsalz mit dem 0,4fachen der zur Neutralisation der Alkalität notwendigen stöchiometrischen Menge $H_2SO_4$ (als 25gew%ige wäßrige Lösung) teilneutralisiert wurde.

Das Endprodukt ist bei 20 °C eine leicht trübe, niedrigviskose Flüssigkeit mit einem Trübungspunkt von 32 °C (gemessen in einer 1 %igen wäßrigen Lösung gemäß DIN 53917).

Der pH-Wert einer 1 %igen wäßrigen Lösung beträgt 6,6; man erhält eine OH-Zahl (korr.) von 89. Der Q-Wert beträgt 2450. Tabelle 1 enthält die Homologenverteilung der hergestellten Alkoholalkoxylate.

Bei der Prüfung der biologischen Abbaubarkeit erreichten die hergestellten Alkoholalkoxylate den im Rahmen der 29-tägigen Versuchsdauer geforderten Grenzwert eines Abbaugrades größer gleich 60 Gew% innerhalb von 10 Tagen nach Überschreiten der 10 Gew%-Grenze (= Beginn der Abbauphase) und wurden deshalb als biologisch leicht abbaubar eingestuft. Bei einer Prüfkonzentration von 7 mg/L waren die Alkoholalkoxylate nach 22 Tagen zu 100 Gew% abgebaut. Tabelle 2 enthält die Daten zur biologischen Abbaubarkeit der Alkoholalkoxylate.

Tabelle 1

| Homologenverteilung des Fettalkohol-Ethylenoxid (EO)/Propylenoxid (PO)-Blockalkoxylats | | | | | | | | | | | |
|---|---|---|---|---|---|---|---|---|---|---|---|
| EO + PO-Zahl | 1 | 2 | 3 | 4 | 5 | 6 | 7 | 8 | 9 | 10 | 11 |
| Gew.-% | 1,8 | 2,3 | 2,8 | 3,9 | 6,4 | 10,5 | 15,1 | 17,5 | 17,1 | 13,3 | 9,2 |

Tabelle 2

| Biologische Abbaubarkeit des Fettalkohol-EO/PO-Blockalkoxylats | |
|---|---|
| Prüfkonzentration | 7 [mg/L] |
| Biologischer Abbau nach 6 d | 35 [%] |
| nach 14 d | 86 [%] |
| nach 22 d | 100 [%] |
| nach 29 d | 100 [%] |

Beispiel 2

1. Stufe:

In einem rührbaren Druckreaktor wurden 194 g (1,00 mol) eines $C_{12/14}$-Alkohol-Gemisches vorgelegt. Nach üblicher Spülung des Reaktionsraumes mit Stickstoff wurde auf 90 °C aufgeheizt und 1 Stunde unter vermindertem Druck gerührt, um den Wassergehalt auf <0,10 Gew% zu senken.
Anschließend wurden 2,9 g des Katalysators 2 zugesetzt und bei 150 - 160 °C in einem Zeitraum von 3 Stunden insgesamt 97 g Ethylenoxid (2,2 mol) zudosiert. Zur Abreaktion des Epoxids wurde 1 Stunde bei 150 - 160 °C nachgerührt.

2. Stufe:

Zu dem aus der 1. Stufe im Reaktor vorliegenden $C_{12/14}$-Alkohol-Ethoxylat wurden 2,0 g einer 50 %igen wäßrigen NaOH-Lösung zugegeben und anschließend 2 Stunden bei 90 °C unter einem verminderten Druck von 20 mbar getrocknet.
Anschließend wurden bei 120 - 130 °C insgesamt 232 g Propylenoxid (4,0 mol) in einem Zeitraum von 5 Stunden zudosiert. Zur Abreaktion des Epoxids wurde 1 Stunde bei 135 °C nachgerührt.
Anschließend wurde mit Isononansäure neutralisiert.

Das Endprodukt ist bei 20 °C eine leicht trübe, niedrigviskose Flüssigkeit mit einem Trübungspunkt von 27 °C in Butyldiglykol/Wasser (gemessen nach DIN 53917). Der pH-Wert einer 1 %igen wäßrigen Lösung beträgt 7,1; man erhält eine OH-Zahl (korr.) von 112. Der Q-Wert beträgt 1220. Tabelle 3 enthält die Homologenverteilung der hergestellten Alkoholalkoxylate.

Bei der Prüfung der biologischen Abbaubarkeit erreichten die hergestellten Alkoholalkoxylate den im Rahmen der 29-tägigen Versuchsdauer geforderten Grenzwert eines Abbaugrad größer gleich 60 Gew% innerhalb von 10 Tagen nach Überschreiten der 10 Gew%-Grenze (= Beginn der Abbauphase) und wurden deshalb als biologisch leicht abbaubar eingestuft. Bei einer Prüfkonzentration von 7 mg/L waren die Alkoholalkoxylate nach 29 Tagen zu 100 Gew% abgebaut. Tabelle 4 enthält die Daten zur biologischen Abbaubarkeit der Alkoholalkoxylate.

Bei Katalysator 2 handelt es sich um eine Verbindung der Formel (II), wobei

$R^3$ iso-$C_{12}$-Alkenyl,
$R^4$ $C_{12/14}$-Alkyl,
n 0,
M Ca und
z 1 ist

und dieses Alkenylbernsteinsäurehalbester-Calciumsalz mit dem 0,4fachen der zur Neutralisation der Alkalität notwendigen stöchiometrischen Menge $H_2SO_4$ (als 25gew%ige Lösung) teilneutralisiert wurde.

Tabelle 3

| Homologenverteilung des Fettalkohol Ethylenoxid (EO)/Propylenoxid (PO)-Blockalkoxylats | | | | | | | | | | | |
|---|---|---|---|---|---|---|---|---|---|---|---|
| EO + PO-Zahl | 1 | 2 | 3 | 4 | 5 | 6 | 7 | 8 | 9 | 10 | 11 |
| Gew.-% | 7,0 | 9,5 | 11,2 | 11,8 | 13,6 | 14,0 | 13,2 | 9,4 | 6,1 | 2,7 | 1,7 |

Tabelle 4

| Biologische Abbaubarkeit des Fettalkohol-EO/PO-Blockalkoxylats | |
|---|---|
| Prüfkonzentration | 7 [mg/L] |
| Biologischer Abbau nach 6 d | 29 [%] |
| nach 14 d | 64 [%] |
| nach 22 d | 90 [%] |
| nach 29 d | 100 [%] |

**Patentansprüche**

1. Alkoxylierungsprodukt-Mischungen der Formel I mit enger Alkoxylverteilung,

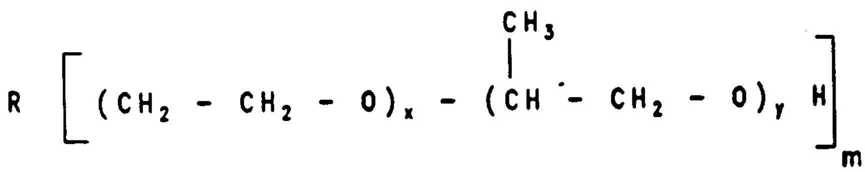

(I)

wobei R der Rest einer organischen Verbindung mit mindestens einem aktiven Wasserstoffatom, m eine ganze Zahl von 1 bis zur Zahl der aktiven Wasserstoffatome der organischen Verbindung ist, x und y jeweils die durchschnittliche Molzahl an Ethylenoxid- bzw. Propylenoxideinheiten sind und unabhängig voneinander eine ganze Zahl von 2 bis 10 sind und die einzelnen Alkoxylierungseinheiten der Alkoxylierungsprodukt-Mischung jeweils maximal einen Alkoxylierungsgrad von 18 Gew% aufweisen.

2. Alkoxylierungsprodukt-Mischungen nach Anspruch 1, dadurch gekennzeichnet, daß in Formel I m = 1 ist und x und y unabhängig voneinander eine Zahl von 2 bis 6 sind.

3. Alkoxylierungsprodukt-Mischungen nach Anspruch 1 oder 2, dadurch gekennzeichnet, daß in Formel I m = 1 ist, x und y unabhängig voneinander sind und x eine Zahl von 2 bis 6 und y eine Zahl von 2 bis 4 ist.

4. Alkoxylierungsprodukt-Mischungen nach einem oder mehreren der Ansprüche 1 bis 3, dadurch gekennzeichnet, daß R in Formel I eine geradkettige oder verzweigte, gesättigte oder ungesättigte $C_8$-$C_{18}$-Alkoxygruppe oder Gemische davon bedeutet.

5. Alkoxylierungsprodukte nach einem oder mehreren der Ansprüche 1 bis 4, dadurch gekennzeichnet daß in Formel I m = 1 ist, x und y unabhängig voneinander sind und x eine Zahl von 2 bis 6 und y eine Zahl von 2 bis 4 ist und R eine einwertige, geradkettige oder verzweigte, gesättigte oder ungesättigte $C_8$-$C_{18}$-Alkoxygruppe oder Gemische davon bedeutet.

6. Verfahren zur Herstellung von Alkoxylierungsprodukt-Mischungen nach einem oder mehreren der Ansprüche 1 bis 5, umfassend die Verfahrensschritte

- Umsetzung einer organischen Verbindung mit mindestens einem aktiven Wasserstoffatom mit Ethylenoxid in einem Molverhältnis von 2 bis 10 mol Ethylenoxid pro mol aktiven Wasserstoffs in der zu ethoxylierenden Verbindung in Gegenwart von mindestens einem Erdalkalimetallsalz der Alkyl- oder Alkenylbernsteinsäuremonoester der nachstehenden Formeln (II) und (III) als Katalysator,

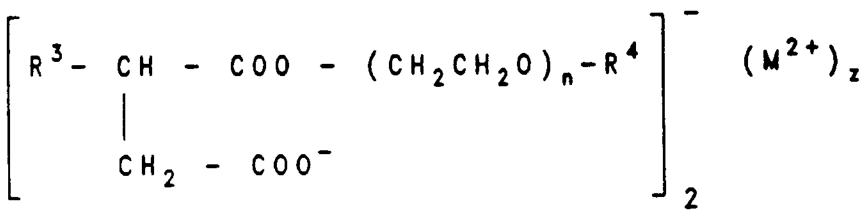

(II)

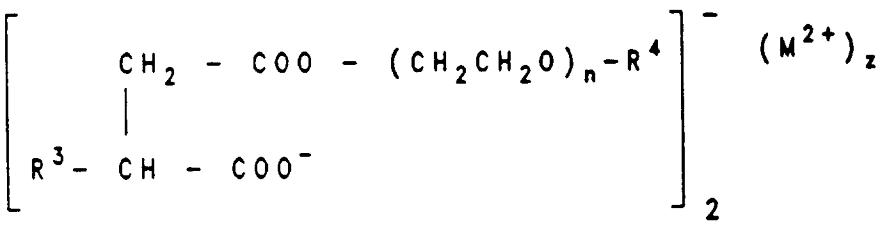

(III)

wobei

$R^3$ $C_8$ bis $C_{30}$-Alkyl oder $C_8$ bis $C_{30}$-Alkenyl,

n eine Zahl von 0 bis 6,

$R^4$ $C_1$ bis $C_{18}$-Alkyl oder $C_3$ bis $C_{18}$-Alkenyl oder Wasserstoff, wenn n = 1 oder >1 ist,

M Ba, Ca oder Sr und

z eine Zahl von 0,9 bis 1,8 ist,

- anschließend Zugabe einer Base zu der erhaltenen Ethoxylatprodukt-Mischung, und Zugabe von Propylenoxid.

**7.** Verfahren nach Anspruch 6, dadurch gekennzeichnet, daß Ethylenoxid und Propylenoxid jeweils in einer Menge von 2 bis 6 mol pro mol aktiven Wasserstoffs in der zu alkoxylierenden organischen Verbindung eingesetzt werden.

**8.** Verfahren nach Anspruch 6 oder 7, dadurch gekennzeichnet, daß der Ethoxylatprodukt-Mischung zur weiteren Katalyse als Base eine wäßrige Natriumhydroxid-Lösung zugesetzt wird.

**9.** Verfahren nach einem der Ansprüche 6 bis 8, dadurch gekennzeichnet, daß das nach Zugabe der Base zu der Ethoxylat-Produktmischung erhaltene Gemisch bei einer Temperatur von 70 bis 150 °C und unter einem verminderten Druck von 10-100 mbar getrocknet wird.

**10.** Verwendung der Alkoxylierungsprodukt-Mischung nach einem oder mehreren der Ansprüche 1 bis 5 als schwachschäumende Tenside, Dispergier- oder Netzmittel.

Europäisches Patentamt

# EUROPÄISCHER RECHERCHENBERICHT

Nummer der Anmeldung
EP 95 10 9012

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | DE-A-15 93 043 (MARLES-KUHLMANN-WYANDOTTE)<br>* Ansprüche; Beispiele *<br>--- | 1-5,10 | C07C43/11<br>C07C41/03<br>C11D1/722 |
| X | DE-A-21 40 010 (BASF)<br>* Seite 1, Zeile 1 - Zeile 5; Ansprüche; Beispiele *<br>--- | 1-5,10 | |
| X | US-A-4 207 421 (M. SCARDERA ET AL)<br>* das ganze Dokument *<br>--- | 1-5,10 | |
| A | US-A-4 223 164 (KANG YANG ET AL)<br>* Abbildungen 6,7 *<br>--- | 1-5 | |
| A | EP-A-0 337 239 (HENKEL)<br>* Seite 3, Zeile 51 - Seite 4, Zeile 45; Ansprüche *<br>--- | 6-9 | |
| P,D,A | DE-A-43 41 576 (HOECHST)<br>* das ganze Dokument *<br>----- | 1-10 | |

RECHERCHIERTE SACHGEBIETE (Int.Cl.6)
C07C
C11D

Der vorliegende Recherchenbericht wurde für alle Patentansprüche erstellt

| Recherchenort | Abschlußdatum der Recherche | Prüfer |
|---|---|---|
| DEN HAAG | 17. Oktober 1995 | Wright, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................
& : Mitglied der gleichen Patentfamilie, übereinstimmendes Dokument